# EUROPEAN PATENT APPLICATION

(11) **EP 2 586 875 A1**
(43) Date of publication of application: **01.05.2013**
(21) Application number: 11306394.5
(22) Date of filing: 27.10.2011
(51) Int. Cl.: C12Q 1/68

(54) **Non-invasive method for diagnosing and monitoring glioma**

(71) Applicant: Institut du Cerveau et de la Moelle Epiniere-ICM, 75013 Paris (FR); Centre National de la Recherche Scientifique - CNRS, 75016 Paris Cedex 16 (FR); Assistance Publique Hôpitaux De Paris, 75004 Paris (FR); Université Pierre et Marie Curie - Paris 6, 75005 Paris (FR); INSERM (Institut National de la Santé et de la Recherche Médicale), 75013 Paris (FR)
(72) Inventor: Boisselier, Blandine, 91600 Savigny Sur Orge (FR); Marie, Yannick, 91210 Draveil (FR); Sanson, Marc, 75006 Paris (FR)
(74) Representative: Cabinet Plasseraud

(57) **Abstract**

The present invention relates to a method for diagnosing a glioma and/or predicting the outcome of a glioma in a patient, comprising the step of extracting a DNA population having a length inferior to 500bp from a plasma sample obtained from said patient. The invention also relates to the use of isocitrate dehydrgenase 1 (IDH1) as a plasma biomarker.

SEQUENCE LISTING

<110>  Institut du Cerveau et de la Moelle

<120>  Non-invasive method for diagnosing and monitoring glioma

<130>  BEP110735EP

<160>  8

<170>  PatentIn version 3.5

<210>  1
<211>  20
<212>  DNA
<213>  Artificial Sequence

<220>
<223>  primer

<400>  1
cggtcttcag agaagccatt                                                   20


<210>  2
<211>  24
<212>  DNA
<213>  artificial

<220>
<223>  primer

<400>  2
cacatacaag ttggaaattt ctgg                                              24


<210>  3
<211>  19
<212>  DNA
<213>  artificial

<220>
<223>  primer


<220>
<221>  locked
<222>  (7)..(7)
<223>  locked nucleic acid in which the ribose ring is locked by a
       methylene bridge connecting the 2\222-O atom and the 4\222-C atom

<220>
<221>  locked
<222>  (10)..(10)
<223>  locked nucleic acid in which the ribose ring is locked by a
       methylene bridge connecting the 2\222-O atom and the 4\222-C atom

<220>
<221>  locked
<222>  (13)..(13)
<223>  locked nucleic acid in which the ribose ring is locked by a
       methylene bridge connecting the 2\222-O atom and the 4\222-C atom

<220>
<221>  locked

<222>  (16)..(16)
<223>  locked nucleic acid in which the ribose ring is locked by a
       methylene bridge connecting the 2\222-O atom and the 4\222-C atom

<400>  3
tcataggtca tcatgctta                                                    19


<210>  4
<211>  19
<212>  DNA
<213>  artificial

<220>
<223>  primer


<220>
<221>  locked
<222>  (7)..(7)
<223>  locked nucleic acid in which the ribose ring is locked by a
       methylene bridge connecting the 2\222-O atom and the 4\222-C atom

<220>
<221>  locked
<222>  (10)..(10)
<223>  locked nucleic acid in which the ribose ring is locked by a
       methylene bridge connecting the 2\222-O atom and the 4\222-C atom

<220>
<221>  locked
<222>  (13)..(13)
<223>  locked nucleic acid in which the ribose ring is locked by a
       methylene bridge connecting the 2\222-O atom and the 4\222-C atom

<220>
<221>  locked
<222>  (16)..(16)
<223>  locked nucleic acid in which the ribose ring is locked by a
       methylene bridge connecting the 2\222-O atom and the 4\222-C atom

<400>  4
tcataggtcg tcatgctta                                                    19


<210>  5
<211>  21
<212>  DNA
<213>  artificial

<220>
<223>  primer

<400>  5
agtggatggg taaaacctat c                                                 21


<210>  6
<211>  24
<212>  DNA
<213>  primer

<400>  6
aatcacatta ttgccaacat gact                                              24

<210>  7
<211>  414
<212>  PRT
<213>  Homo sapiens

<400>  7

<210>  8
<211>  452
<212>  PRT
<213>  Homo sapiens

<400>  8

## Description

### FIELD OF THE INVENTION

The present invention relates to methods for diagnosing and monitoring glioma.

### BACKGROUND OF THE INVENTION

Gliomas, or brain tumors, can be classified into the following grades, according to the classification of the World Health Organization (1).
- Low-grade gliomas [WHO grade II] are well-differentiated (not anaplastic); these are not benign but still portend a better prognosis for the patient.
- High-grade [WHO grade III-IV] gliomas are undifferentiated or anaplastic; these are malignant and carry a worse prognosis. Grade IV gliomas include primary glioblastomas (or primary GBM) and secondary glioblastomas (or secondary GBM), the latter deriving from lower grades gliomas.

Despite intensive therapies, including surgery, radiotherapy, and chemotherapy, the outcome of glioma patients remains dismal, with a median overall survival time for patients with the most aggressive subtype (glioblastoma [GBM]) reaching only 15 months (2). Current research efforts focused on identifying genetic alterations implicated in glioma might help to define subgroups of patients with different prognoses and different responses to specific treatments.

Identified for the first time in 2008 (3) the *IDH1* gene mutation has rapidly been recognized as one of the most powerful independent prognostic factor ever described in glioma patients. Indeed, patients whose tumors harbored *IDH1* mutation survived much longer than patients with a wild-type *IDH1* glioma, whatever the grade (4). The *IDH1* gene, which encodes for the isocitrate dehydrogenase 1, is mutated in one third of gliomas, the percentage of mutated tumors reaching 75% and 50% in WHO grade II gliomas and grade III gliomas respectively. Mutations in the *IDH1* gene always affect the codon 132, which in more than 90% of cases result in a substitution of arginine by histidine, p.Arg132His (p.R132H or IDH1R132H) (4-6). Apart from his major prognostic impact, the *IDH1* mutation has also a profound diagnostic importance (6). Even if a few studies have reported the frequency of *IDH1* mutations in other solid malignancies, only rare mutations were detected in prostate carcinomas and colorectal cancers (< 3%). The *IDH1* mutation is almost exclusively found in gliomas, thus constituting an excellent identifying biomarker of this pathology.

Histological analysis of tumor tissue obtained from biopsy or surgery is currently the gold standard for cancer diagnosis. However these procedures are only performed once and can not be repeated during patients treatment and follow up. Moreover, in certain glioma patients the brain tumor is non-operable, either because of the localization of the tumor, or because of the general condition of the patient.

To complement or possibly replace the initial histological diagnosis, many groups have focused on the identification of reliable biomarkers in bodily fluids (7). For glioma patients, no plasma biomarker has been identified so far. The analysis of DNA freely circulating in the blood is one of the most promising approaches (8). To date, only three papers have reported the presence of plasma DNA in glioma patients (9-11). These studies showed that patients with glioma have large amounts of circulating DNA in the blood and that epigenetic alterations such as *MGMT* promoter methylation, can be detected using this DNA. However, the inventors have found that this DNA is highly contaminated by leukocyte DNA and is not suitable for detecting mutations associated with glioma.

Hence, there is still an unmet need in the art for plasma biomarkers for glioma patients and for non-invasive methods for diagnosing glioma.

### SUMMARY OF THE INVENTION

The inventors have shown that a population of DNA molecules having a length inferior to 500bp could be isolated from the plasma of patients suffering from glioma. They have further demonstrated that this DNA could be used to detect mutations in genes associated with glioma, such as the *IDH1* gene.

Thus, the invention relates to a method for diagnosing a glioma and/or predicting the outcome of a glioma in a patient, comprising the step of extracting a DNA population having a length inferior to 500bp from a plasma sample obtained from said patient.

The invention also relates to a method for monitoring the response to treatment in a patient suffering from glioma comprising the step of extracting a DNA population having a length inferior to 500bp from a plasma sample obtained from said patient.

The invention also relates to the use of IDH1 as a plasma biomarker for glioma.

### DETAILED DESCRIPTION OF THE INVENTION

Thus, the invention relates to a method for diagnosing a glioma and/or predicting the outcome of a glioma in a patient, comprising the step of extracting a DNA population having a length inferior to 500 bp from a plasma sample obtained from said patient.

As used herein, the term "glioma" has its general meaning in the art. It encompasses all brain tumors, irrespective of the grade or histological type. Therefore, the term "glioma" encompasses astrocytomas, oligodendromas, and glioblastomas.

As used herein, the term "diagnosing" has its general meaning in the art. It refers to the process of attempting to determine and/or identify a possible disease or disorder.

The method for diagnosing glioma according to the invention can be carried out in combination with any method commonly used for diagnosing glioma, such as Magnetic Resonance Imaging (MRI), resection of the tumor followed by histological analysis, or any other suitable method known in the art.

As used herein, the expression "predicting the outcome" refers to the assessment of the severity of the glioma in a patient and to the prediction of the progression free survival rate (also called PFS, corresponding to the time to progression) and the overall survival rate (such as the percentage of patients surviving after 6 months, 1 year, 2 years, 5 years etc) associated with patients suffering from a specific type of glioma. The expression "predicting the outcome" also refers to the disease follow-up, corresponding both to the natural evolution of the disease and to the response to treatments.

As used herein, the term "patient" denotes a mammal, such as a rodent, a feline, a canine, or a primate. Preferably, a patient according to the invention is a human.

### DNA population

As used herein, the term "DNA" has its general meaning in the art and refers to desoxynucleic acid. The population of DNA according to the invention is low molecular weight DNA, i.e. the DNA molecules have a length inferior to 500 base pairs (bp), preferably inferior to 450bp, even more preferably inferior to 400bp, 350bp, 300bp, 250bp, 200bp. In a preferred embodiment, the length of the DNA molecules in the isolated DNA population ranges from 100bp to 250 bp, preferably from 110 bp to 240 bp, even more preferably from120 bp to 230 bp, 130 bp to 220 bp, 140 bp to 210 bp, 150 bp to 200 bp.

The length of the DNA population can be evaluated by any suitable method in the art.

As used herein, the expression "length of the DNA" refers to the length of the majority of the DNA molecules in a given population. The skilled person will easily understand that there can exist, among an isolated DNA population having a certain length, certain contaminating DNA molecules having a different length. Typically, more than 80% by weight, preferably more than 85%, even more preferably more than 90%, 95%, 96%, 97%, 98%, 99%, 99.5%, 99.6%, 99.7%, 99.8%, 99.9% of the molecules of said DNA population have a specified length.

Without wishing to be bound by theory, it is believed that the DNA population having a length inferior to 500bp comprises free circulating DNA fragments which have been released by the tissues into the blood circulation. In particular, it is believed that the population of DNA having a length inferior to 500bp comprises fragments of DNA originating from the tumor. Said DNA fragment therefore contain the molecular signature of the tumor and can be useful for diagnosing and/or classifying gliomas.

In one embodiment of the invention, the method further comprises the step of verifying the length of the extracted free circulating DNA by electrophoresis.

Advantageously, this step serves the purpose of verifying that the DNA population obtained corresponds to DNA fragments released from the tumor rather than contaminant DNA originating from white blood cells. This step enhances the specificity of the method.

Typically, the length of the DNA population can be assessed by chromatography techniques such as, but not limited to, electrophoresis. By way of example, verification of the length of the molecules of the DNA population can be carried out with the Agilent High Sensitivity DNA kit using a 2100 Bioanalyzer (Agilent Technologies).

### Mutations

The inventors have found that mutations associated with glioma could be detected in the DNA population obtained from patients suffering from glioma.

In particular, they have shown that mutations in the *IDH1* gene could be detected.

In one embodiment, the extracted DNA is then analyzed to identify the presence or absence of a mutation in a gene associated with glioma.

Typically, said gene associated with glioma can be the isocitrate dehydrogenase 1 gene (*IDH1*) or the isocitrate dehydrogenase 2 gene *(IDH2).*

Indeed, mutations in isocitrate dehydrogenase genes are known to be specifically found in the tumoral DNA of glioma patients.

Furthermore, mutations in isocitrate dehydrogenase genes are associated with good prognosis, whatever the grade of the glioma, with the exception of gangliogliomas in which mutations in the isocitrate dehydrogenase gene are associated with a bad prognosis (12).

Therefore, the invention relates to a method for diagnosing a glioma in a patient comprising the steps of:
- extracting a DNA population having a length inferior to 500bp from a plasma sample obtained from said patient:
- analysing said DNA population to identify the presence or absence of a mutation in the *IDH1* or *IDH2* gene;
wherein a mutation is associated with glioma.

As used herein, the term "mutation" has its general meaning in the art. It refers to a change in amino acid sequence when compared to a sequence of reference, such as a so-called wild-type sequence, and encompasses mutations by insertion, deletion, and/or substitution.

The term "mutation" also applies a mutant gene in which the mutation results in a modified amino acid in the encoded protein. Mutations that result in a modified amino acid can be identified according a standard nomenclature: for example R132H designates a mutant in which the wild-type R residue at codon 132 of the wild-type protein is replaced by an H residue.

The common amino acid at codon 132 of IDH1 and codon 172 of IDH2 in healthy tissues in arginine (R). Mutant codons have been with substitutions of histidine (H), serine (S), and cysteine (C), leucine (L), and glycine (G) of IDH1 codon132 and methionine (M), lysine (K), and glycine (G) of codon 172 of IDH2

In one embodiment, said mutation is a mutation in the *IDH1* gene.

In a preferred embodiment, said mutation is R132H in the *IDH1* gene.

The step of analyzing the DNA population to identify the presence or absence of mutation in a gene associated with glioma can be performed by any suitable method known in the art. Preferably, sensitive detection techniques, such as PCR in tandem with a number of different downstream assays for detecting somatic mutations, are used. These detection techniques include, but are not limited to RFLP analysis, MALDI-TOF (matrix-assisted laser-desorption time-of-flight) genotyping, high resolution melting curve analysis or direct sequencing for detection of mutations by Sanger sequencing or pyrosequencing.

Since the mutated alleles are in minority in the DNA population, techniques that preferentially amplify the mutated allele are preferred.

Thus, in one embodiment, the step of analyzing said DNA population to identify the presence or absence of a mutation in the *IDH1* or *IDH2* gene is performed by COLD-PCR and/or digital PCR.

COLD-PCR (or co-amplification at lower denaturation temperature Polymerase Chain Reaction) is a modified Polymerase Chain Reaction (PCR) protocol that enriches variants alleles from a mixture of wild-type and mutation-containing DNA.

This approach uses the critical temperature (Tc) during the PCR process in order to enrich mutations at any position of the amplified sequence. Indeed, a single nucleotide mismatch anywhere along the a double-stranded DNA sequence generates a small change in the melting temperature for that sequence, with mutated sequences melting at a lower temperature than wild-type sequences (13).

Suitable COLD-PCR protocols include, but are not limited to, the protocol described in Boisselier et al. (14).

Typically, in one embodiment, the step of analyzing the DNA population to identify the presence or absence of a mutation in the *IDH1* gene comprises performing COLD-PCR using the forward and reverse primers having the sequence as set forth in SEQ ID No: 1 and SEQ ID No:2 respectively.

Digital PCR is a refinement of the PCR methods in which the sample is separated into a large number of wells so that each well contains statistically 0 or 1 DNA molecule, and the amplification reaction is carried out in each well individually. This technique enables quantitative detection of mutated alleles and is highly sensitive.

Typically, in one embodiment, the step of analyzing the DNA population to identify the presence or absence of a mutation in the *IDH1* gene is performed by digital PCR using the forward and reverse primers having the sequence as set forth in SEQ ID No:3 and SEQ ID No:4 and the locked nucleic acid probes having the sequence as set forth in SEQ ID No:5 and SEQ No:6.

Locked nucleic acid probes or "LNA" probes are oligonucleotides containing one or several "locked" nucleotides. The ribose moiety of an LNA nucleotide is modified with an extra bridge connecting the 2' oxygen and 4' carbon. This bridge "locks" the ribose in the 3'-endo (North) conformation. The resulting oligonucleotide has an increases melting temperature and hybridization mismatches are thus avoided. LNA probes are therefore used in order to increase the specificity of the reaction.

In a preferred embodiment the step of analyzing the DNA population to identify the presence or absence of a R132H mutation in the *IDH1* gene is carried by
- performing a COLD PCR step as follows: 1 cycle of 96°C, 10 min; 20 cycles of 95°C, 15 sec; 60°C, 30 sec, then 30 cycles of 81°C, 15 sec; 60°C, 30 sec., in a final mixture containing LightCycler^{®}480 HRM Master (Roche Applied Science), 0.25mM forward and reverse primers (respectively SEQ ID No:1 and SEQ ID No:2), 3mM MgCl₂, and 5µL of DNA template in order to yield PCR amplicons (step a);
- diluting at 1/1,000,000 of the amplicons of step a with distilled water (step b);
- performing a digital PCR step under the following QPCR cycling conditions: 96°C, 1 min; 45 cycles of 95°C, 1 sec; 62°C, 25 sec on a 1536-well plate, each well containing 1µL of a master mix containing RealTime Ready Probes Master 2X (Roche Applied Science), 300 nM forward and reverse primers (SEQ ID No:3 and SEQ ID No:4 respectively), 200 nM wild type and mutant LNA probes (SEQ ID No:5 and SEQ ID No:6 respectively), and diluted PCR amplicons.

In one embodiment, the invention relates to a method for diagnosing and/or predicting the outcome of a patient suffering from glioma as described above, wherein a mutation is indicative of high grade glioma having a tumor volume superior to 3.5 cm³, preferably superior to 3 cm³, even more preferably superior to 2.5 cm³ or 2 cm³.

As used herein, the expression "tumor volume" refers to the volume of a tumor as measured by conventional imaging techniques using constrast enhancement. Typically, the size of a tumor is measured by magnetic resonance imaging as described in the Examples below.

### Plasma preparation

As used herein, the term "plasma" has its general meaning in the art. It refers to the liquid component of blood from which the blood cells have been removed. Typically, a plasma sample is obtained by submitting a blood sample to centrifugation and collecting the supernatant.

Typically, the plasma sample can be obtained by:
- subjecting a blood sample obtained from said patient to a first centrifugation step;
- subjecting the supernatant obtained from the first centrifugation step to a second centrifugation step;
- collecting the supernatant.

Typically, the first centrifugation step is carried out at 800 to 2,000 g, preferably 900 to 1,500g, even more preferably 950g to 1,200g, 980g to 1,100g, even more preferably about 1,000 g. Typically, said first centrifugation step is carried out at 4°C for 10 to 20 minutes, preferably for 15 minutes.

In a preferred embodiment said first centrifugation step is carried out at 4°C, for 15 minutes at 1,000 g.

Typically, the second centrifugation step is carried out at 8,000 to 16,000 g, preferably 9,000 to 11,000 g, even more preferably 10,000 g. Typically, said second centrifugation step is performed at room temperature for 5 to 15 minutes.

In a preferred embodiment said second centrifugation step is carried out at room temperature, for 5 minutes at 10,000 g.

In one embodiment, the plasma sample is obtained by:
- subjecting a blood sample obtained from said patient to a first centrifugation step for 15 minutes at 1000 g at 4°C;
- subjecting the supernatant obtained from the first centrifugation step to a second centrifugation step for 5 minutes at 10,000 g at room temperature;
- collecting the supernatant.

Optionally, the plasma sample may be stored at a temperature ranging from -80°C to +4°C before the extraction step.

Typically, the plasma sample can be stored at +4°C or -20°C or -80°C before the circulating DNA fragments are extracted.

In a preferred embodiment, the plasma sample is stored at -80°C.

The step of extracting free circulating DNA can be performed by any suitable method for extracting small DNA fragments having a length inferior to 500bp.

Typically, the extraction step can be performed by chromatography using an appropriate silica membrane.

For example, the extraction step may be performed using QIAamp Circulating Nucleic Acid kit (Qiagen reference 55114), according to the manufacturer's instructions.

### Prognostic methods and patient follow-up, based on the amount of DNA

In one embodiment of the invention, the method for diagnosing a glioma and/or predicting the outcome of glioma comprises the step of quantifying the amount of free circulating DNA, wherein the amount of DNA is correlated with the volume of the tumor.

Indeed, the inventors has demonstrated that, for high grade gliomas, significantly higher amounts of DNA having a length inferior to 500bp were found in the plasma of patients suffering from tumors having a volume above 10cm³ compared to patients suffering from a tumor having a volume under 10 cm³.

Without wishing to be bound by theory, it is believed that tumoral DNA is released into the plasma of patients suffering from a high grade glioma (grade 3 or grade 4 gliomas), due to the disruption of the blood-brain barrier. Therefore, the greater the tumor volume, the greater the amount of tumor-derived DNA extracted from the plasma will be.

This only appears to apply for high grade tumors (i.e gliomas of grade 3 or 4). Indeed, in low grade glioma, the blood-brain barrier is still intact and the amount of DNA having a length inferior to 500bp in the plasma seems to be essentially the same as in healthy patients.

Typically, the amount of DNA having a length inferior to 500bp, in particular ranging from 150bp to 200bp, can be assessed by chromatography techniques such as, but not limited to, high sensitivity electrophoresis. By way of example, verification of the length of the molecules of the DNA population can be carried out with the Agilent High Sensitivity DNA kit using a 2100 Bioanalyzer (Agilent Technologies).

In one embodiment the invention therefore relates to a method for predicting the outcome of a patient suffering from glioma as defined above, further comprising the step of quantifying the amount of DNA having a length inferior to 500bp, wherein low amounts of DNA are indicative of a good prognosis.

As used herein, the term "good prognosis" has its general meaning in the art. It refers to a higher survival rate of patients in which a mutation has been detected, compared to patients suffering from a glioma of the same grade and having a tumor of the same length.

Conversely, the invention also relates to a method for predicting the outcome of a patient suffering from glioma as defined above, further comprising the step of quantifying the amount of DNA having a length inferior to 500bp, wherein low amounts of DNA are indicative of a good prognosis.

Typically, said amount of DNA having a length inferior to 500bp can be compared to an average amount obtained in the plasma of a healthy population.

Typically, the amount of DNA in a plasma sample shall be deemed to be elevated if it is superior to 1.5 ng/mL, preferably superior to 2 ng/mL, even more preferably 3 ng/mL, 4 ng/mL, 5 ng/mL, 6 ng/mL, 7 ng/mL, 8 ng/mL, 9 ng/mL, 10 ng/mL, 15 ng/mL, 20 ng/mL, 25 ng/mL, 30 ng/mL.

The invention also relates to a method for predicting the outcome of a patient suffering from glioma comprising the steps of:
- extracting a DNA population having a length inferior to 500bp from a first plasma sample obtained from a patient at t1,
- measuring the amount of DNA having a length inferior to 500bp in said first plasma sample,
- extracting a DNA population having a length inferior to 500bp from a plasma sample obtained from a patient at t2,
- measuring the amount of DNA having a length inferior to 500bp is said second plasma sample;
and wherein a decrease in the amount of DNA having a length inferior to 500bp in the second sample as compared to the amount of DNA having a length inferior to 500bp in the first sample is indicative of a decrease of the tumor size between t1 and t2.

Conversely, an increase in the amount of DNA having a length inferior to 500bp in the second sample as compared to the amount of DNA having a length inferior to 500bp in the first sample is indicative of a increase of the tumor size between t1 and t2 and/or an increase in the grade of the tumor.

Typically, t1 and t2 can be any consecutive time points, separated by an interval of one or several days, one or several months, or one or several years.

In one embodiment, when the method of the invention is used to monitor the response to therapy, t1 can be prior to therapy and t2 during or following therapy. Alternatively, t1 can be a first time point during therapy, and t2 a later time point during therapy or following therapy.

In another embodiment, when the method of the invention is used to monitor the progression of the disease after surgery, t1 and t2 can be two consecutive time points following removal of the tumor by surgery.

According to this embodiment, when the patient suffering from a glioma has been identified as harbouring a mutation in the tumor and/or in the plasma, excision of the tumor should lead to the disappearance of mutated DNA in the plasma. Plasma samples can then be obtained at regular intervals in order to verify that no DNA harbouring a mutation, i.e. no DNA originating from a new tumor, re-appears in the plasma of the patient over time.

### Prognostic methods and patient follow-up, based on the presence of a mutation

In another aspect, the invention relates to a method for predicting the outcome of a patient suffering from a high grade glioma comprising the steps of:
- extracting a DNA population having a length inferior to 500bp from a plasma sample obtained from said patient:
- analyzing said DNA population to identify the presence or absence of a mutation in the *IDH1* or *IDH2* gene;
wherein a mutation is associated with a good prognosis.

Indeed, it has been shown that a mutation in the IDH1 or IDH2 gene within the tumor is associated with a good prognosis.

Since elevated amounts of DNA are released from the tumor into the plasma in the case of high grade glioma patients, the presence of a mutation in the DNA population extracted from a plasma sample of a patient suffering from a high grade glioma is associated with good prognosis, compared to a patient suffering from a high grade glioma of comparable tumor size (and therefore containing a comparable amount of tumor-derived DNA in the plasma) who does not present a mutation in the plasma DNA.

According to one embodiment, the method can further comprise determining a ratio of mutant allele to wild-type allele and comparing said ratio to predetermined cut-off value.

Said ratio of mutant allele to wild-type allele can be calculated by any suitable method. Typically, when the detection of the mutation is carried out by COLD-PCR, followed by digital PCR, the ratio can be calculated as the number of informative wells with mutant probe divided by the number of informative wells with the wild-type probe x100.

Advantageously, comparing said ratio to a cut-off value increases the specificity of the method, by elimination of false-positives.

In another embodiment, the invention relates to a method for predicting the outcome of a patient suffering from glioma comprising the steps of:
- extracting a DNA population having a length inferior to 500bp from a first plasma sample obtained from a patient at t1,
- extracting a DNA population having a length inferior to 500bp from a first plasma sample obtained from a patient at t2,
- analyzing both DNA populations to identify the presence or absence of a mutation in the *IDH1* or *IDH2* gene,
- determining a ratio of the mutant allele to wild-type allele in both DNA populations,
wherein a decrease in said ratio between t1 and t2 is indicative of a reduction in the size of the tumor.

### Biomarker

In another aspect, the invention relates to the use of IDH1 as a plasma biomarker for glioma.

In another aspect, the invention also relates to the use of IDH2 as a plasma biomarker for glioma.

The term "plasma biomarker" refers to a DNA molecule which can be detected in a plasma sample of patient and whose mutation is predictive or denotes a condition of the patient from which it was obtained.

The invention will be further illustrated by the following examples. However, these examples should not be interpreted in any way as limiting the scope of the present invention.

### FIGURE LEGENDS

**Figure 1****: Comparison of the capillary electrophoresis profiles obtained with single (upper panel) and double centrifugations (lower panel) procedures.** After extraction, plasma DNA was run on an Agilent High Sensitivity DNA kit.
**Figure 2****: Representative electrophoregrams obtained with DNA extracted from 1 mL (A) and 4 mL (B) of plasma**
**Figure 3****: A.** Plasma DNA concentration in healthy controls and in low and high grade gliomas patients. **B.** Plasma DNA concentrations in healthy controls and in high grade gliomas patients stratified according to tumor volume. HC: healthy controls, LGG: low grade gliomas patients, HGG: high grade gliomas patients. * *p<* 0.05; ** *p<* 0.01
**Figure 4****: A.** Receiver Operating Characteristic (ROC) curve for *IDH1* mutation in plasma sample. **B.** Percentage of mutant DNA evidenced by digital PCR. Data from 13 samples in *IDH1* wild type glioma patients and 25 samples from *IDH1* mutant glioma patients are included.

### EXAMPLES

### Methods

### Patients

The patients treated in the Neuro Oncology department of the Pitié Salpêtrière Hospital were selected according to the following criteria: 1) histologic diagnosis of glioma according to the World Health Organization WHO, 2) *IDH1* status previously determined by Sanger sequencing on tumor DNA, 3) written informed consent before enrolment in the study. Healthy controls were recruited within laboratory and hospital staff. Blood samples were collected when patients underwent brain MRI scans during their standard clinical follow-up.

### Tumor volume evaluation on MRI scans

Magnetic resonance imaging (MRI) scans were conducted on a 1.5 Teslas apparatus in the department of neuroradiology of the Pitié Salpêtrière hospital. MRI scans were performed within a median of 5 days from blood sampling and were available for all patients. Each MRI has been performed according to a standardized protocol that included pre- and post-contrast enhanced T1-weighted images in multiple planes, as well as Fluid Attenuated Inversion Recovery (FLAIR) or T2-weighted sequences. The formula used to estimate tumor volume was the modified ellipsoid volume equation, as follows: (A x B x C)/2 where A, B and C represent the three largest orthogonal diameters of the enhancing lesion. Such diameters were manually measured on MRI films with calipers. Tumor volume calculation was based on the contrast enhanced volume for high grade gliomas and the T2/FLAIR hyperintensity volume for low grade gliomas. Non-injected T1-weighted images were used to differentiate tumor enhancement from hemorrhage.

### DNA extraction

Blood samples were collected in EDTA-coated tubes and centrifuged immediately at 1,000g for 15 minutes at 4°C. Plasma was carefully collected and centrifuged once again at 10,000g for 5 minutes at room temperature. Plasma was immediately aliquoted and stored at -80°C until use. A total 50µL of DNA was extracted from 4mL of plasma using QIAamp^{®} Circulating Nucleic Acid kit (Qiagen) and a vacuum chamber (QIAvac24 Plus, Qiagen,) according to the manufacturer's instructions. Once extracted, DNA samples were treated with RNase A, 20mg/mL (Invitrogen) to discard RNA carriers, and purified using NucleoSpin^{®} Extract II (Macherey Nagel). Samples were then run on an Agilent High Sensitivity DNA kit using a 2100 Bioanalyzer (Agilent Technologies), allowing both determinations of DNA length and DNA concentration by determination of the area under the curve.

### Detection of IDH1 mutation by digital PCR in plasma samples

The detection of *IDH1* mutation in plasma samples was performed following a 2 steps procedure: selective amplification of mutant DNA by COLD (co-amplification at lower denaturation temperature) PCR and characterization by digital PCR.

Plasma DNA was first amplified by a run of COLD PCR, as previously described (Boisselier et al.). COLD PCR cycling conditions on LightCycler^{®}480 (Roche Applied Science) were as follows: 1 cycle of 96°C, 10 min; 20 cycles of 95°C, 15 sec; 60°C, 30 sec, then 30 cycles of 81°C, 15 sec; 60°C, 30 sec. COLD PCR assays contained final reagent concentrations as follows: LightCycler^{®}480 HRM Master (Roche Applied Science), 0.25mM forward and reverse primers (Invitrogen), 3mM MgCl₂, and 5µL of DNA template (Table 1). PCR amplicons were diluted at 1/1,000,000 with distilled water and were then characterized by digital PCR.

For the digital PCR step, PCR products of each sample were assayed on a 1536 well plate. Briefly, a master mix PCR (RealTime Ready Probes Master 2X, Roche Applied Science), 300 nM forward and reverse primers (Eurogentec), 200 nM wild type and mutant LNA probes (Eurogentec), and diluted PCR amplicons (Table 1) were loaded (1µL/well) in a LightCycler^{®}1536 Multiwell Plate, using the Bravo Automated Liquid Handling Platform (Agilent Technologies). QPCR cycling conditions on LightCycler^{®}1536 (Roche Applied Science) were as follows: 96°C, 1 min; 45 cycles of 95°C, 1 sec; 62°C, 25 sec.

The heatmaps were analyzed using LightCycler^{®} 1536 software. For each individual specimen, the percentage of mutant DNA (mutant amplification ratio) was defined as the number of informative wells with mutant probe / number of informative wells with wild-type probe.

**Table 1: Primers used for the COLD PCR and the digital PCR steps. For LNA probes, base in bold corresponds to the locked nucleic acids.**

| | Primer | | Amplicon Length |
|---|---|---|---|
| | | CGGTCTTCAGAGAAGCCATT | |
| COLD PCR | Forward | (SEQ ID No :1) | 172 |
| | | CACATACAAGTTGGAAATTTCTGG | |
| | Reverse | (SEQ ID No :2) | |
| Digital PCR | Mutant LNA probe | TCATAGGTCATCATGCTTA | |
| | | (SEQ ID No :3) | 78 |
| | Wild Type LNA probe | TCATAGGTCGTCATGCTTA | |
| | | (SEQ ID No :4) | |
| | | AGTGGATGGGTAAAACCTATC | |
| | Forward | (SEQ ID No :5) | |
| | | AATCACATTATTGCCAACATGACT | |
| | Reverse | (SEQ ID No :6) | |

### Statistical analysis

The statistical analysis was performed using GraphPad 5.0 software. The Mann Whitney test was used to compare plasma DNA concentration and mutant amplification ratio between patients groups. Receiver-operating characteristic (ROC) curve was used to assess the pertinence of using the percentage of mutant DNA in plasma as a diagnostic tool for glioma detection. A p-value < .05 was considered significant.

### Results

### Patients characteristics

A total of 80 patients with gliomas and 31 healthy controls were enrolled, providing a total of 121 collected plasma samples. Characteristics of both glioma patients and healthy controls are summarized in Table 2.

**Table 2: Population characteristics. n: number of subjects**

| | **Gliomas patients** | **Healthy controls** |
|---|---|---|
| n | 80 | 31 |
| Median age [range] | 48 [25-67] | 47 [24-64] |
| Sex ratio | 1.4 (47/33) | 0.6 (12/19) |

### DNA extraction from plasma samples

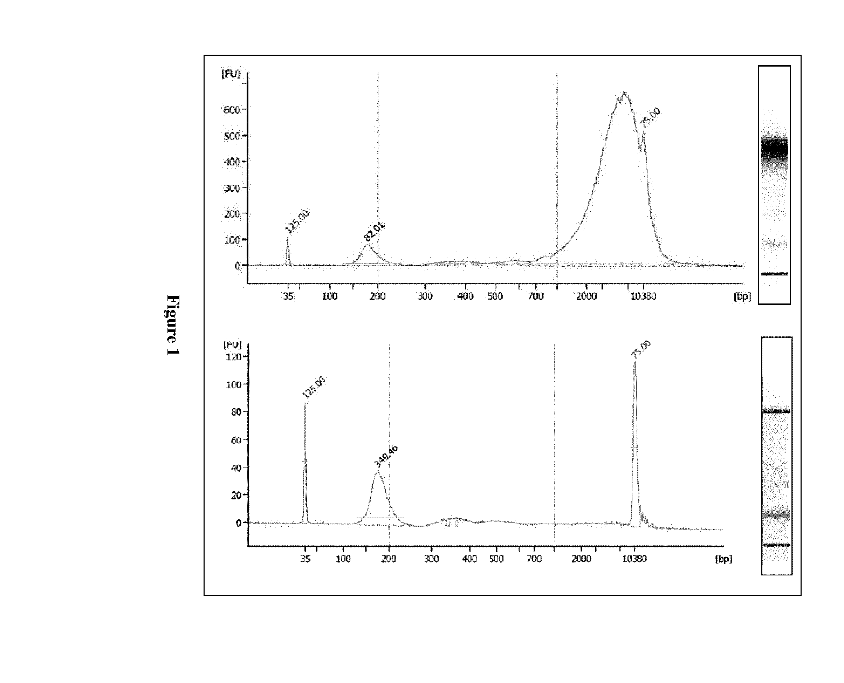

As a first step, a comparison between double and single plasma centrifugation was made. The quality of plasma DNA was then controlled by capillary electrophoresis (Agilent High Sensitivity DNA Kit). As compared to single centrifugation, double centrifugation removed the high molecular weight DNA (derived from leukocytes lysis) and allowed a clear visualization of small size DNA (ssDNA, also called small length DNA) (**Figure 1**).

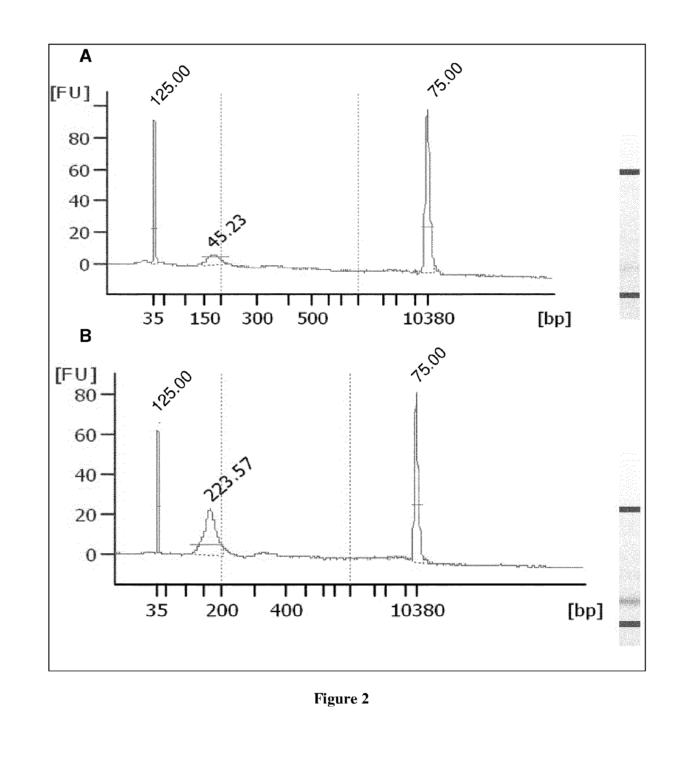

In order to determine the volume of plasma required to ensure an efficient extraction of tumor DNA, the concentrations of ssDNA obtained after extraction of 1 mL and 4 mL of plasma were determined and compared in 11 high grade glioma patients with a voluminous tumor. In all cases, extractions from 1 mL of plasma yielded to a very small or undetectable amount of ssDNA (**Figure 2A**), whereas 4 mL of plasma led to a significant ssDNA amount (**Figure 2B**) in 9 out of 11 cases. For all the following experiments, DNA was systematically extracted from 4 mL of plasma.

### DNA concentrations in plasma

Our results showed that all plasma samples analyzed in this study contained DNA with a size ranging from 150pb to 200pb. Taken as a whole group, the median concentration of plasma DNA in glioma patients (1.2 ng/mL; range 0.1-50.3 ng/mL) was not statistically different from the median DNA concentration in healthy controls plasmas (1.2 ng/mL; range 0.1-6.6 ng/mL),

Samples were then stratified according to tumor grade and volume. The 28 low grade gliomas were divided into 3 groups according to the tumor volume (V) measured on FLAIR MRI sequence: group A (V< 2.5 cm³), group B (2.5< V< 22.5 cm³) and group C (V> 22.5 cm³) (Table 3). Similarly, the 52 plasma samples collected from patients with high grade gliomas were divided into 3 groups according to the tumor enhancement volume (V) measured on T1 gadolinium MRI sequence: group D (no contrast enhancement), group E (V< 10 cm³) and group F (V> 10 cm³).

**Table 3: Median plasma DNA median concentration (ng/mL) according to tumor volume**

| | | **n** | **Tumor volume (V)** | **Median plasma DNA concentration (ng/mL)** | **Range (ng/mL)** |
|---|---|---|---|---|---|
| **Low grade** | | | | | |
| **gliomas** | **All** | **28** | | **0.9** | **0.0-3.0** |
| | Group A | 7 | <2.5 cm³ | 1.0 | 0.2-3.0 |
| | Group B | 11 | 2.5 cm³<V<22.5 cm³ | 0.7 | 0.0-1.7 |
| | Group C | 10 | >22.5 cm³ | 0.9 | 0.1-3.0 |
| **High grade** | | | | | |
| **gliomas** | **All** | **52** | | **1.5** | **0.1-50.3** |
| | Group D | 23 | No contrast enhancement | 0.9 | 0.1-3.5 |
| | Group E | 15 | V<10 cm³ | 1.6 | 0.4-24.9 |
| | Group F | 14 | >10 cm³ | 14.0 | 0.6-50.3 |
| **Controls** | | 31 | | 1.2 | 0.1-6.6 |

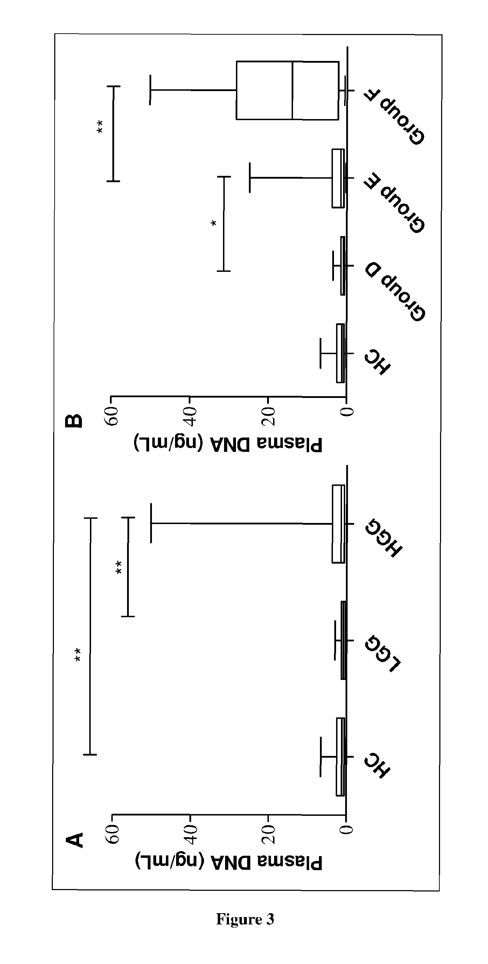

As shown in Table 3, plasma DNA concentrations in low-grade glioma patients were similar to healthy controls (median concentrations: 0.9 vs., 1.2 ng/mL, respectively, *p*=0.16) (Figure 3A). Furthermore, plasma DNA concentration was not correlated with tumor volume in low grade gliomas (median plasma DNA concentrations: 1.0; 0.7 and 0.9 ng/mL for groups A, B, and C, respectively, *p*= 0.28). In contrast, in high grade gliomas, plasma ssDNA concentration was significantly higher in patients than in healthy controls (Figure 3A, 1.5 vs. 1.2 ng/mL, respectively, *p*= 0.0045), and plasma ssDNA concentration increased with tumor volume (Figure 3B): median plasma DNA concentration was 14.0 ng/mL for tumor volumes > 10 cm³ (group F), compared to 1.6 ng/mL for tumor volumes < 10 cm³ (group E, *p*= 0.0083).

### Detection of IDH1 mutation in plasma DNA

Thirty-nine patients (25 with R132H *IDH1* mutated glioma, 14 with *IDH1* wild type glioma) were further analyzed for the detection of *IDH1* mutation on the ssDNA extracted from plasma (Table 4).

Given the expected high contamination by non neoplastic DNA, both COLD PCR -for the selective amplification of mutant DNA- and digital PCR -a highly sensitive approach to detect *IDH1* mutation- were combined. Digital PCR conditions were considered valid for a given sample when the total number of amplifications was comprised between 100 and 1200 out of the 1536 wells of the IDH1 wild type assay.

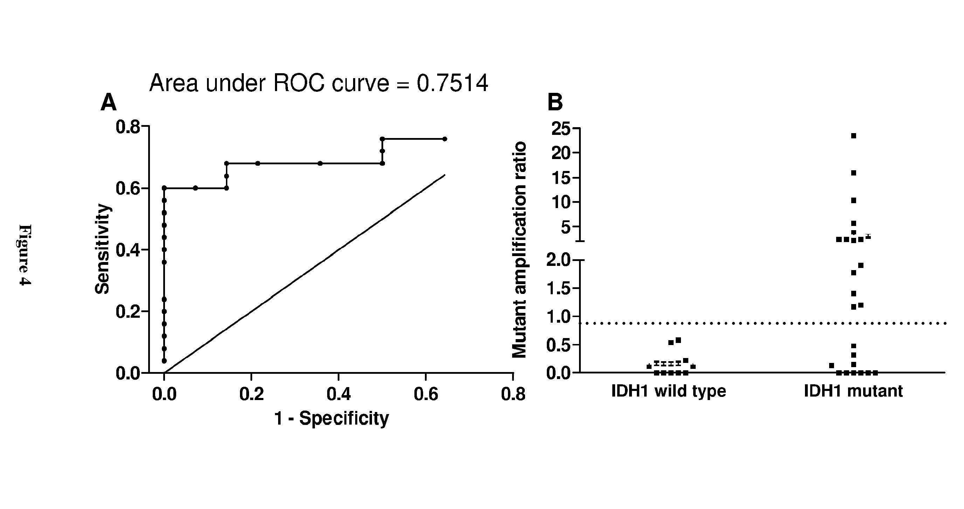

The mutant amplification ratio (cf supra: number of informative wells with mutant probe/ number of informative wells with wild type probe) ranged from 0% to 23.5%. The mutant amplification ratio was 0.16% [0-0.58%] for the 14 *IDH1* wild type glioma patients vs. 3.2% [0-23.5%] for the 25 *IDH1* mutant glioma patients (*p*= 0.0096) (Table 4). The area under the ROC curve assessing percentage of mutant DNA was 0.7514 (Figure 4A). For obtaining the required specificity of 100% (95%; CI 77%-100%), the highest sensitivity was 60% (95%; CI 39%-79%) corresponding to a threshold of positivity of 0.875 for mutant amplification ratio (Figure 4B). Under these conditions, the mutation was detected in plasma DNA of 15 out of 25 patients (60%) harboring an *IDH1* mutated tumor, and in none of the 14 patients harboring an *IDH1* negative tumor (Figure 4B).

Out of 39 patients, 12 had a low grade glioma and 8 of them had the R132H *IDH1* mutation (Table 5). R132H *IDH1* mutation was detected in only three of them (37.5%) on plasmatic ssDNA. In all of them, the FLAIR volume was >22.5 cm³ (46.7; 96.0 and 174.7 cm³; group C). Among the 27 patients with a high-grade glioma, the mutation was detected in the plasma of 12 out of 17 patients (70.6%) with R132H *IDH1* mutated tumor. The detection rate of *IDH1* mutation in plasma was correlated to the enhancing tumor volume. IDH1 mutation was not detected in the plasma of the only patient exhibiting no contrast enhancement (group D). The mutation was detected in the plasma of 6/10 (60%) patients in group E (enhancing V<10 cm3), and in 6/6 (100%) patients in group F (V>10 cm3). The smallest tumor enhancement volume for which the *IDH1* mutation was detected in plasma was 0.15 cm³. When analyzing the high grade glioma patients as a whole group, we found that all mutant tumors with an enhancing tumor volume higher than 3.5 cm³ (9/9) were detected by digital PCR in plasma.

**Table 4: Detection of IDH1 mutation by digital PCR in plasma DNA**

| | *IDH1* wild type gliomas | *IDH1* mutant gliomas |
|---|---|---|
| N | 14 | 25 |
| % DNA mutant [range] | 0.16 [0-0.58] | 3.2 [0-23.5] |

**Table 5: Detection of IDH1 mutation in plasma in a cohort of 39 glioma patients**

| | | **FLAIR or T1 post contrast volume** | **n** | **Wild type *IDH1* patients** | **Mutant *IDH1* patients** |
|---|---|---|---|---|---|
| **Low grade gliomas** | **All** | | **12** | **4/4** | **3/8 (37.5%)** |
| | Group A | <2.5 cm³ | - | - | - |
| | Group B | 2.5 cm³<V<22.5 cm³ | 3 | 1/1 | 0/2 (0%) |
| | Group C | >22.5 cm³ | 9 | 3/3 | 3/6 (50%) |
| **High grade gliomas** | **All** | | **27** | **10/10** | **12/17 (70.6%)** |
| | Group D | <0 cm³ | 1 | 0/0 | 0/1 (0%) |
| | Group E | 0 cm³<V<10 cm³ | 14 | 4/4 | 6/10 (60%) |
| | Group F | >10 cm³ | 12 | 6/6 | 6/6 (100%) |

### REFERENCES

1. Louis DN, Ohgaki H, Wiestler OD, Cavenee WK, Burger PC, Jouvet A, et al. The 2007 WHO classification of tumours of the central nervous system. Acta Neuropathol. 2007;114:97-109.
2. Stupp R, Hegi ME, Mason WP, van den Bent MJ, Taphoorn MJ, Janzer RC, et al. Effects of radiotherapy with concomitant and adjuvant temozolomide versus radiotherapy alone on survival in glioblastoma in a randomised phase III study: 5-year analysis of the EORTC-NCIC trial. Lancet Oncol. 2009;10:459-66.
3. Parsons DW, Jones S, Zhang X, Lin JC, Leary RJ, Angenendt P, et al. An integrated genomic analysis of human glioblastoma multiforme. Science. 2008;321:1807-12.
4. Sanson M, Marie Y, Paris S, Idbaih A, Laffaire J, Ducray F, et al. Isocitrate dehydrogenase 1 codon 132 mutation is an important prognostic biomarker in gliomas. J Clin Oncol. 2009;27:4150-4.
5. Ichimura K, Pearson DM, Kocialkowski S, Backlund LM, Chan R, Jones DT, et al. IDH1 mutations are present in the majority of common adult gliomas but rare in primary glioblastomas. Neuro Oncol. 2009;11:341-7.
6. Yan H, Parsons DW, Jin G, McLendon R, Rasheed BA, Yuan W, et al. IDH1 and IDH2 Mutations in Gliomas. N Engl J Med. 2009;360:765-73.
7. Ludwig JA, Weinstein JN. Biomarkers in cancer staging, prognosis and treatment selection. Nat Rev Cancer. 2005;5:845-56.
8. Schwarzenbach H, Hoon DS, Pantel K. Cell-free nucleic acids as biomarkers in cancer patients. Nat Rev Cancer. 2011;11:426-37.
9. Balana C, Ramirez JL, Taron M, Roussos Y, Ariza A, Ballester R, et al. 06-methyl-guanine-DNA methyltransferase methylation in serum and tumor DNA predicts response to 1,3-bis(2-chloroethyl)-1-nitrosourea but not to temozolamide plus cisplatin in glioblastoma multiforme. Clin Cancer Res. 2003;9:1461-8.
10. Lavon I, Refael M, Zelikovitch B, Shalom E, Siegal T. Serum DNA can define tumor-specific genetic and epigenetic markers in gliomas of various grades. Neuro Oncol. 2010;12:173-80.
11. Weaver KD, Grossman SA, Herman JG. Methylated tumor-specific DNA as a plasma biomarker in patients with glioma. Cancer Invest. 2006;24:35-40.
12. Horbinski C, Kofler J, Yeaney G, Camelo-Piragua S, Venneti S, Louis DN, et al. Isocitrate dehydrogenase 1 analysis differentiates gangliogliomas from infiltrative gliomas. Brain Pathol. 2011.
13. Li J, Wang L, Mamon H, Kulke MH, Berbeco R, Makrigiorgos GM. Replacing PCR with COLD-PCR enriches variant DNA sequences and redefines the sensitivity of genetic testing. Nat Med. 2008;14:579-84.
14. Boisselier B, Marie Y, Labussiere M, Ciccarino P, Desestret V, Wang X, et al. COLD PCR HRM: a highly sensitive detection method for IDH1 mutations. Hum Mutat. 2010;31:1360-5.

## Claims

1. Method for diagnosing a glioma and/or predicting the outcome of a patient suffering from a glioma, comprising the step of extracting a DNA population having a length inferior to 500bp, preferably ranging between 150bp and 200bp, from a plasma sample obtained from said patient.

2. Method according to claim 1, further comprising the step of verifying the length of the DNA fragments in the extracted DNA population by electrophoresis.

3. Method for diagnosing a glioma according to claim 1 or 2, said method comprising the steps of:
- extracting a DNA population having a length between inferior to 500bp from a plasma sample obtained from said patient;
- analyzing said DNA population to identify the presence or absence of a mutation in the isocitrate dehydrogenase 1 (*IDH1)* or isocitrate dehydrogenase *(IDH2)* gene;
wherein a mutation is associated with glioma.

4. Method according to claim 3, wherein the presence of a mutation is indicative of high grade glioma having a tumor volume superior to 3.5cm³.

5. Method according to any of claims 3 or 4, wherein said mutation is R132H in the *IDH1* gene.

6. Method according to any of claims 3 to 5, wherein the step of analyzing said DNA population to identify the presence or absence of a mutation in the *IDH1* or *IDH2* gene is performed by co-amplification at lower denaturation temperature polymerase chain reaction (COLD-PCR) and/or digital PCR.

7. Method according to claim 6, wherein the step of analyzing the DNA population to identify the presence or absence of a R132H mutation in the *IDH1* gene comprises carrying out:
- a Co-amplification at Lower Denaturation temperature Polymerase Chain Reaction (COLD-PCR) reaction using forward and reverse primers having the sequence as set forth in SEQ ID No: and SEQ ID No:2 respectively;
- followed by a digital PCR reaction using forward and reverse primers having the sequence as set forth in SEQ ID No:3 and SEQ ID No:4 respectively and locked nucleic acid (LNA) probes having the sequence as set forth in SEQ ID No:5 and SEQ ID No:6.

8. Method according to any of the preceding claims, wherein the plasma sample is obtained by:
- subjecting a blood sample obtained from said patient to a first centrifugation step;
- subjecting the supernatant obtained from the first centrifugation step to a second centrifugation step;
- collecting the supernatant.

9. Method according to claim 8, wherein the first centrifugation step is carried out at 1,000 g, for 10 minutes and the second centrifugation step is carried out at 10, 000g for 5 minutes.

10. Method according to any of the preceding claims, further comprising the step of quantifying the amount of DNA having a length inferior to 500bp, and wherein elevated amounts of DNA are indicative of a poor prognosis.

11. Method for predicting the outcome of a patient suffering from glioma comprising the steps of:
- extracting a DNA population having a length inferior to 500bp from a first plasma sample obtained from a patient at t1,
- measuring the amount of DNA having a length inferior to 500bp in said first plasma sample,
- extracting a DNA population having a length inferior to 500bp from a second plasma sample obtained from a patient at t2,
- measuring the amount of DNA having a length inferior to 500bp is said second plasma sample;
and wherein a decrease in the amount of DNA having a length inferior to 500bp in the second sample as compared to the amount of DNA having a length inferior to 500bp in the first sample is indicative of a decrease of the tumor size between t1 and t2.

12. Method for predicting the outcome of a patient suffering from a high grade glioma, comprising the steps of:
- extracting a DNA population having a length inferior to 500bp from a plasma sample obtained from said patient:
- analyzing said DNA population to identify the presence or absence of a mutation in the *IDH1* or *IDH2* gene;
wherein a mutation is associated with a good prognosis.

13. Method for predicting the outcome of a patient suffering from glioma comprising the steps of:
- extracting a DNA population having a length inferior to 500bp from a first plasma sample obtained from a patient at t1,
- extracting a DNA population having a length inferior to 500bp from a second plasma sample obtained from a patient at t2,
- analyzing each DNA population to identify the presence or absence of a mutation in the *IDH1* or *IDH2* gene,
- determining a ratio of the mutant gene to wild-type gene in each DNA population,
wherein a decrease in said ratio at t2 as compared to t1 is indicative of a reduction in the size of the tumor.

14. Use of IDH1 and/or IDH2 as a plasma biomarker for glioma.
